# EUROPEAN PATENT APPLICATION

(11) **EP 3 309 251 A1**
(43) Date of publication of application: **18.04.2018**
(21) Application number: 16807639.6
(22) Date of filing: 13.06.2016
(51) Int. Cl.: C12N 15/09, A61K 39/00, A61K 39/015, A61K 39/02, A61K 39/04, A61K 39/12, A61P 31/04, A61P 31/12, A61P 35/00, C07K 14/115

(54) **HUMAN PARAINFLUENZA TYPE 2 VIRUS VECTOR AND VACCINE**

(30) Priority: 12.06.2015 JP 2015119097; 28.03.2016 JP 2016063315
(71) Applicant: Mie University, Tsu-shi, Mie 514-8507 (JP); BioComo Inc., Mie-gun, Mie 510-1233 (JP)
(72) Inventor: NOSAKA, Tetsuya, Tsu-shi Mie 514-8507 (JP); TSURUDOME, Masato, Tsu-shi Mie 514-8507 (JP); FUKUMURA, Masayuki, Mie-gun Mie 510-1233 (JP); OHTSUKA, Junpei, Mie-gun Mie 510-1233 (JP); YUDA, Masao, Tsu-shi Mie 514-8507 (JP); IWANAGA, Shiroh, Tsu-shi Mie 514-8507 (JP)
(74) Representative: Keller, Günter
(86) International application number: PCT/JP2016/067516
(87) International publication number: WO 2016/199936

(57) **Abstract**

The present invention relates to: a virus vector, which can effectively transfer a macromolecular antigenic peptide into target cells while maintaining a three-dimensional structure that is required for functioning as an antigen; and a vaccine utilizing the vector. Specifically, disclosed are: a virus vector, in which a nucleic acid encoding an antigenic polypeptide is integrated immediately 5' upstream of HN gene of an F gene-defective Paramyxoviridae virus gene, wherein the antigenic polypeptide is expressed as a fusion protein of 130 or more amino acid residues, fused with a TM sequence and/or a CT sequence derived from the virus.

## Description

### Technical Field

### [Related Application]

This specification contains the contents described in the specifications of Japanese Patent Application No. 2015-119097 (filed on June 12, 2015) and Japanese Patent Application No. 2016-63315 (filed on March 28, 2016), which the right of priority of the present application is based on.

### [Technical field]

The present invention relates to a non-transmissible human parainfluenza virus type 2 vector and a vaccine prepared by utilizing the vector.

### Background Art

For preparation of an efficient neutralizing antibody-inducing recombinant vaccine, it is suitable to use the full length of an envelope protein (RSV F, Ebola GP, etc.) having a membrane fusion activity as a vaccine antigen. However, it has been reported that the protein changes its three-dimensional structure in pre- and postfusion with a target cell or the like; a prefusion protein having a structure before fusion with a cell induces an effective neutralizing antibody, the protein has an unstable structure in a prefusion state, and attempts have been made to stabilize the protein structure in a prefusion state and use it as a vaccine antigen (Non patent Literatures 1 to 4).

RS virus (RSV) is a virus having an envelope, classified as the genus Pneumovirus of the family Paramyxovirus, and having a globose with a diameter of 80 to 350 nm or a filament shape, and it has major subtypes A and B and causes respiratory infections. The initial infection of RSV exhibits various symptoms including mild symptoms such as flu-like symptoms to lower respiratory tract diseases such as severe bronchiolitis or pneumonia. Maternal antibodies against RSV are present, but they gradually decrease and are not detected seven months after birth. An initial infection is apparent and high risk of developing a lower respiratory tract disease and an infection within six months after birth becomes the severest. An epidemiological research (in 2009) reported that the number of yearly infant inpatients aged 5 years or younger was 75 to 125 thousand in US, 80 thousand in five European countries and 30 to 80 thousand in Japan; the number of elderly inpatients aged 65 years was 200 thousand in US, 200 thousand in five European countries and 140 thousand in Japan.

No vaccine effective for RSV infection control is present, and there exists only symptomatic treatment by palivizumab (Synagis) of an anti RSV humanized monoclonal antibody. A preventive effect of palivizumab is expected by intramuscular injection at a dose of 15 mg/kg per one month before the start of RSV prevalence to a prevalence period. However, the humanized monoclonal antibody is quite expensive and insurance covered subjects in Japan are limited to premature infants, children with a chronic lung disease and Down's syndrome children. The high cost of the antibody makes it difficult to expand patients intended for administration.

Therefore, there is a strong need for development of an inexpensive vaccine for RSV infection control. However, the past vaccine disaster described below caused a significant delay in developing a vaccine compared to vaccines for other infectious diseases.

In the years 1965 to 1966, an inactivated vaccine (named as Lot 100) prepared by treating RSV with formalin in the same manner as influenza or polio vaccines was used together with an aluminum adjuvant for clinical tests wherein inoculation was carried out on infants with no RSV infection history. RSV natural infection after the vaccine inoculation caused a tragic result, in which an admission rate in the vaccine inoculation group was 16 times higher than the non-inoculation group (80% of infants in the vaccine inoculation group were hospitalized) and two died (RSV infection severity ERD: enhanced respiratory disease). Since then, the investigation to determine the cause of ERD has been made intensively.

Infiltration of eosinophils or neutrophils into the lung is not observed in bronchiolitis caused by ordinary RSV. However, in the infant death caused by RSV natural infection after the inoculation of formalin-inactivated RSV vaccine (Lot 100), remarkable infiltration of eosinophils, neutrophils, etc. into the lung and excessive induction of T-helper cell type 2 (Th-2) by an increase of IL-4/IL-5 cytokine production were observed. At that time, it was reported that an increase of antibody titer against RSV was observed, but induction of antibodies having a high neutralizing activity titer against RSV was low. It is reported that F protein or one of membrane proteins of RSV is important as a vaccine antigen, but it is also understood that F protein alters its three-dimensional conformation depending on the environment. It has been reported by the animal tests that formalin treatment not only alters the three-dimensional structure of RSV F, but also greatly increases carbonyl content in protein and induces Th2 reaction; as a result, it is highly possible to cause ERD (Non Patent Literature 5). This literature describes that thermally treated RSV (only the three-dimensional structure was altered) vaccine caused a lower neutrophil infiltration to the lung tissue than formalin treated RSV vaccine, and it is considered that ERD induction of formalin-treated RSV vaccine is not merely dependent on the alteration of the three-dimensional structure of an antigen.

For neutralizing antibody induction of RSV, F protein as a membrane protein of RSV is important as an antigen. F protein is translated as F0 or a protein precursor. F0 has two furin cleavage sites present therein, and through maturation process, it is cleaved into F1 and F2, which are crosslinked by S-S bond (mature F protein). Furin cleavage deletes 27 polypeptides called as p27. It is known that the mature F protein forms a trimer on infected cells and on a virus envelope and it takes two different three-dimensional structures, prefusion F and postfusion F, depending on the environment. F protein before RSV-cell fusion usually takes prefusion F structure, but this structure is thermodynamically unstable and will be easily changed to thermodynamically stable postfusion F structure by cell fusion, heating, a denaturant, deletion of a transmembrane sequence responsible for anchoring of F protein to a cell and an envelope. Three-dimensional structures of prefusion F and postfusion F are found by X-ray crystallographic analysis, and antibodies bound by each of them and their binding sites have been reported in detail (Non Patent Literature 1).

Palivizumab, a monoclonal antibody, binds to a site called as site II of RSV F protein. The sequence domain thereby maintains the three-dimensional structure in both prefusion F and postfusion F, and palivizumab binds to F protein with both structures and exhibits neutralizing activity.

Meanwhile, an antibody has been reported, which binds to an amino acid sequence called siteφ (zero) of an antigen epitope having the three-dimensional structure of prefusion F. A domain near the epitope takes a bending structure composed of two β sheets and four α helixes in prefusion F, while the domain in postfusion F takes a linear structure with one α helix structure. As described above, there is a difference in siteφ (zero) structure between prefusion F and postfusion F, and antibodies named as 5C4, Am22 and D25 bind only to the prefusion F structure and do not bind to F protein of postfusion.

Further, it is known that a prefusion F site φ (zero) binding antibody has 10 to 100 times higher neutralizing activity compared to a site II binding antibody, and for effective vaccine development, it is important to develop a vaccine technique that can provide F protein taking a stable prefusion structure by modification as an antigen.

Possible methods for providing prefusion F protein as an antigen include: (i) stabilizing a prefusion F protein structure, (ii) developing an antigen delivery system while the three-dimensional structure of prefusion F is maintained; and a method with a combination of both of the above.

Possible methods for (i) stabilizing the structure include structure-stabilizing modification of prefusion F protein by deletion or substitution of amino acid sites of a portion/both of two furin recognition sites; or by deletion or substitution of p27 peptide located between furin recognition sites. Regarding amino acid substitutions at other sites of RSV F, it is considered that p27-deleted mutant of RSV is stabilized by substituting Asn(N) at position 67 and Ser(S) at position 251 of RSV F by Ile(I) and Pro(P), respectively (Non Patent Literature 4). It is also considered that RSV F is stabilized by substituting both of Ser(S) at position 155 and Ser(S) at position 290 by Cys(C) and providing S-S bond between cysteines (Non Patent Literature 7). Further, it is considered that the structure is stabilized by deleting a portion or the entire of a cell fusion domain located directly after the rear sequence of the two furin recognition sites of F protein and eliminating fusion ability.

When F protein is used as an antigen, soluble F protein (soluble form F protein) wherein a secretion signal sequence present at the N-terminal side is left, and a transmembrane (TM) sequence and a cytoplasmic tail (CT) sequence at the C-terminal side are deleted, is often used. If F protein carrying the sequences is expressed in cells, anchoring in the membrane occurs, thereby making the recovery difficult. Thus, the deletion of the sequences is adopted in order to secrete F protein in a medium for allowing easy recovery. Meanwhile, F protein having TM and CT sequences enables formation of a trimer and is anchored on a cell membrane or a virus envelope, thereby stabilizing the prefusion structure. When F protein having the prefusion structure is used as an antigen, deletion of the TM and CT sequences of F protein leads to easy recovery but an unstable structure. On the other hand, if the TM and CT sequences are left, the structure is stabilized but the recovery becomes difficult.

Then, for (ii), it is possible to take a measure for developing a delivery system, which carries F protein to a recipient in a state where F protein is allowed to bind onto a cell membrane or a virus envelope, a vector, etc. with a stabilized structure while the TM and CT sequences are retained, or a measure wherein the system is combined with (i).

The inventors are developing a method for producing a non-transmitting virus vector carrying an antigen by fusing a foreign antigenic gene with a membrane protein gene of non-transmitting hPIV2 virus vector, and utilizing it as a vaccine; and further, a method for inactivating the virus with β-propiolactone at a lower concentration of 1/10 to 1/100 than the concentration for ordinary treatment to prevent viral replication and utilizing it as a vaccine (Patent Literature 1). These methods have enabled a gene (113 amino acid residues) prepared by fusing 48 amino acids having two tandemly linked versatile influenza antigens M2e with TM and CT sequences of hPIV2 F protein to be cloned into a NotI restriction site 5' upstream of NP gene having a highest level of transcription in F-deficient hPIV2 vector, thereby successfully recovering viruses sufficient to conduct animal tests.

RSV F protein, Ebola virus GP protein, etc. are an antigen having 500 or more amino acid residues, and they require a strict three-dimensional structure to function as an antigen. There is a need for developing a vector and a vaccine capable of delivering these macromolecular proteins while the proteins retain the three-dimensional structures.

### Citation List

### Patent Literature

Patent Literature 1: WO2014/103310

### Non Patent Literature

Non Patent Literature 1: MaLellan, JS. et al., Science: 2013:340, 6136, 1113-1117
Non Patent Literature 2: MaLellan, JS. et al., Science: 2013: 342, 6158-8, 592-598
Non Patent Literature 3: Bale, S. et al., Viruses, 2012: (4), 447-470
Non Patent Literature 4: Krarup, A. et al., Nat.Commun. 2015: 3, 6: 8143-54
Non Patent Literature 5: Moghaddam, A. et al., Nat Med 2006 (12) 905-907
Non Patent Literature 6: Bukreyev, A. et al., Virogy; 2009: 383(2), 348-361
Non Patent Literature 7: Liang, B. et al., J Virol; 2015, (89) : 9499-9510
Non Patent Literature 8: Bernstein, DI. et al., Pediatr. Infect. Dis. J.:2012 (31), 109-114
Non Patent Literature 9: Ynang, CH. et al., Vaccine: 2013: (31), 2822-2827
Non Patent Literature 10: Hara K, et al.: HUMAN GENE THERAPY: 2013(24),683-691
Non Patent Literature 11: Ohtsuka, J. et al., Gene Ther. 2014. 21(8), 775-784
Non Patent Literature 12: Odell, D. et al., J Virol; 1997(71), 7996-8000
Non Patent Literature 13: Ohtaki, N. et al., Vaccine: 2010(28), 6588-6596
Non Patent Literature 14: Zhang, S, et al., Vilology Jounal: 2011(8), 333-339
Non Patent Literature 15: Martinez. O, et al., J Virol: 2013 (87) 3324-3334

### Summary of Invention

### Technical Problem

A problem to be solved by the present invention is to provide a virus vector capable of effectively delivering a macromolecular antigenic peptide (including proteins and peptides) into a target cell while maintaining the three-dimensional structure required for functioning as an antigen; and a vaccine utilizing the vector.

### Solution to Problem

The inventors have found that a macromolecular antigenic peptide having several hundred of amino acid residues can be effectively delivered to a target cell while maintaining its three-dimensional structure by inserting an antigenic peptide gene immediately 5' upstream of HN gene of a non-transmissible Paramyxoviridae virus genome gene lacking a membrane protein gene, and fusing the antigenic peptide and envelope protein TM and CT sequences of a virus vector with each other and expressing the fused resultant.

Specifically, the present invention relates to the following (1) to (9).
(1) A non-transmissible virus vector in which a nucleic acid encoding an antigenic polypeptide is integrated immediately 5' upstream of HN gene of an F gene-defective Paramyxoviridae virus genome gene, wherein the antigenic polypeptide is expressed as a fusion protein of 130 or more amino acid residues, fused with a TM sequence and/or a CT sequence derived from the virus.
(2) The virus vector according to (1), wherein the Paramyxoviridae virus is a non-transmissible human parainfluenza virus type 2.
(3) The virus vector according to (1) or (2), wherein the antigenic polypeptide is expressed on the surface of a vector envelope.
(4) The virus vector according to any one of (1) to (3), wherein the antigenic polypeptide is expressed while maintaining a natural three-dimensional structure or a three-dimensional structure required for a vaccine antigen.
(5) The virus vector according to any one of (1) to (4), wherein the virus has undergone nucleic acid inactivation treatment.
   The nucleic acid inactivation treatment is, for example, a treatment with a low concentration of β-propiolactone, which does not substantially change the virus envelope structure.
(6) The virus vector according to any one of (1) to (5), wherein a TM sequence and/or a CT sequence of an antigenic polypeptide gene or a GPI-like anchor protein is integrated as a nucleic acid substituted by a TM sequence and/or a CT sequence derived from human parainfluenza virus type 2.
(7) The virus vector according to any one of (1) to (6), wherein the antigenic polypeptide is any one or more of:
   an antigenic peptide of a virus selected from influenza viruses including a highly virulent influenza virus, parainfluenza virus type 3, RS virus, Hendra virus, SARS virus, MERS virus, Nipah virus, Lassa virus, dengue virus, West Nile virus, Japanese encephalitis virus, human metapneumovirus, Ebola virus, hantavirus, AIDS virus, hepatitis C virus, hepatitis B virus, rubella virus, rotavirus, norovirus, Crimean-Congo hemorrhagic fever virus, herpesvirus, cytomegalovirus, Zika virus, Marburg virus, HIV and papillomavirus;
   an antigenic peptide of a bacterium selected from the group A beta-hemolytic streptococcus, Mycobacterium tuberculosis, Vibrio cholerae, and mycoplasma;
   an antigenic peptide of plasmodium; and
   an antigenic peptide selected from the group consisting of cancer antigens gp100, MUC1, NY-ESO-1, MelanA/MART1, TRP2, MAGE, CEA, CA125, HER2/neu, WT1, PSA and a neoantigen,
   or fragment(s) thereof.
(8) The virus vector according to (7), wherein the antigenic polypeptide is any one selected from: an antigen having three or more consecutive M2e of influenza virus; RSV F protein or G protein, or a mutant thereof or a fragment thereof; Ebola virus GP protein, or a mutant thereof or a fragment thereof; and malaria CSP protein, or a mutant thereof or a fragment thereof; and Zika virus prM/E protein, or a mutant thereof or a fragment thereof.
   The antigenic polypeptide is integrated into a virus vector in the following forms:
   a) a nucleic acid including an antigenic gene having three or more consecutive M2e of influenza virus and TM and CT sequences of human parainfluenza virus type 2 F protein;
   b) a gene encoding the full length of RSV F protein, or a nucleic acid wherein TM and/or CT sequences of the protein is substituted by TM and/or CT sequences of human parainfluenza virus type 2 F protein, or a nucleic acid encoding a protein substituted by TM and/or CT sequences of human parainfluenza virus type 2 F protein, harboring amino acid mutations for increasing the stability of prefusion F;
   c) a gene encoding F protein having a partial deletion of a cell fusion domain sequence (SEQ ID NO: 5) from the full length of RSV F protein, or a nucleic acid encoding F protein harboring amino acid mutations for increasing the stability of prefusion F, wherein TM and/or CT sequences of the F protein is substituted by TM and/or CT sequences of human parainfluenza virus type 2 F protein;
   d) a gene encoding RSV F protein wherein a p27 peptide sequence between two furin cleavage domains and RR of the first furin cleavage recognition sequence of the p27 are deleted or a mutant thereof, or a gene encoding RSV F protein wherein the first furin cleavage recognition sequence RARR is substituted by QAQQ, or in a mutant thereof, a nucleic acid encoding a protein wherein TM and/or CT sequences of the F protein is substituted by TM and/or CT sequences of human parainfluenza virus type 2 F protein;
   e) a nucleic acid encoding a gene wherein a Fibritin trimerization sequence gene of T4 phage or a GCN trimerization sequence gene is inserted at 3'-terminal side of RSV F extramembranous sequence gene of the nucleic acids described in the above b) to d), or a nucleic acid encoding a gene wherein an amino acid is substituted by Cys for formation of S-S bond for stabilizing trimerization;
   f) in the full length GP protein of Ebola virus or a mutant thereof, a nucleic acid wherein TM and CT sequences of the GP protein are substituted by TM and CT sequences of human parainfluenza virus type 2 F protein or GP protein, or TM and CT sequences of the GP protein are maintained
      (examples of the Ebola virus mutant include mutant GP protein wherein Phe (F) at position 88 and Phe (F) at position 535 in the amino acid sequence (GenBank Accession No. KJ660346) of Ebola virus GP protein are both substituted by Ala(A), mutant GP1 protein wherein a mucin domain sequence located at position 313 and onward from the mutant GP protein is deleted, or mutant GP protein wherein a mucin domain sequence is deleted from the GP sequence);
   g) a nucleic acid encoding: malaria CSP protein, or a protein wherein GPI-type anchor sequence of the protein is substituted by TM and/or CT sequences of human parainfluenza virus type 2 F protein;
   h) a nucleic acid encoding Zika virus prM/E protein; and i) a nucleic acid encoding a protein wherein, of two stem sequences (EH1 and EH2) and two TM sequences (TM1 and TM2) located at the C-terminal region of Zika virus E protein, TM and/or CT sequences of human parainfluenza virus type 2 F protein having a deletion of EH1 and EH2 or a deletion of EH2 are added.
(9) A vaccine comprising the virus vector according to any one of (1) to (8) and a pharmaceutically acceptable carrier.

### Advantageous Effects of Invention

The virus vector of the present invention can deliver a macromolecular peptide (having several hundred or more amino acid residues) as an antigen while the peptide retains the three-dimensional structure. This allows the production of a licensed vaccine effective to RSV and Ebola virus, a plasmodium or Zika virus.

The virus vector of the present invention can be recovered efficiently in a state where an antigenic polypeptide is displayed on a virus particle. Further, the virus vector of the present invention undergoes nucleic acid inactivation by use of a low concentration of β-propiolactone or the like, so virus genome alone is inactivated while the virus envelope protein and loaded antigen maintain the three-dimensional structures. Thus, in the case of a recombinant virus vector with the pre-existence of antibodies, the vector is neutralized by the antibodies and the amount of antigen in a recipient is reduced by gene silencing. However, the vector of the present invention does not have such problem, and it has an advantage that no mutation of antigen gene occurs in a recipient. Further, this vector has cell adsorption properties and immunogenicity similar to those of live viruses, and at the same time, a loaded antigen can maintain the three-dimensional structure of an antigen epitope for inducing an efficient neutralizing antibody. Thus, with no addition of an adjuvant or with addition of a reduced amount thereof, an effective vaccine effect can be expected. From the above advantages, use of the virus vector of the present invention allows the expectation of excellent effects and safety even in humans, and enables the development a licensed vaccine effective to RSV, Ebola virus, malaria or Zika virus.

### Brief Description of Drawings

[Figure 1] Figure 1 shows the construction scheme of 3xM2e gene with TM/CT sequence of hPIV2 F, cloned in an Mlu restriction enzyme site.
[Figure 2] Figure 2 shows: (A) confirmation results of M2e expression in infected cells with recovered vectors; and (B) confirmation results of antigen loading on the vector particles by Western blotting.
[Figure 3] Figure 3 shows the scheme of transfer of RSV virus F antigen.
[Figure 4] Figure 4 shows: (A) the expression of mutant RSV F in infected cells via hPIV2ΔF; and (B) results of mutant expression by Western blotting.
[Figure 5] Figure 5 shows the scheme of transfer of Ebola virus GP antigen.
[Figure 6] Figure 6 shows: (A) the expression of Ebola GP having two introduced amino acid mutations in hPIV2□F vectors, in full-length form of Ebola GP, or Ebola GP fused with hPIV2 TM/CT sequence; and (B) results of Western blotting indicating that GP is incorporated into vector particles.
[Figure 7] Figure 7 shows confirmed results by using GFP fluorescence as an indicator of PIV2 F expression in Vero cells expressing hPIV2 F and parental Vero cells, to see whether infective particles of hPIV2ΔF viruses carrying Ebola GP and GFP protein with introduced amino acid mutations at two sites are produced or not.
[Figure 8] Figure 8 shows results of antibody induction against Ebola GP by administration of the vector into mice.
[Figure 9] Figure 9 shows the scheme of transfer of CSP antigens of Plasmodium yoelli.
[Figure 10] Figure 10 shows results on the expression of CSP antigen confirmed by Wester blotting.
[Figure 11] Figure 11 shows results of antibody induction against CSP by administration of the vector into mice.
[Figure 12] Figure 12 shows the scheme of transfer of Zika virus prM/E antigen.

### Description of Embodiments

The present invention relates to a virus vector wherein a nucleic acid encoding an antigen polypeptide is integrated immediately 5' upstream of HN gene of an F gene-defective Paramyxoviridae virus genome gene, wherein the antigenic polypeptide is expressed as a fusion protein of 130 or more amino acid residues, fused with/substituted by TM and/or CT sequences derived from the virus.

In one aspect, the present invention provides a virus vector in which a gene encoding an antigenic polypeptide is integrated so as to be expressed on a virus surface in the form including fusion with/substitution by TM sequence and/or CT sequence gene of Paramyxoviridae virus F gene, wherein a macromolecular antigenic polypeptide is expressed while maintaining a desired three-dimensional structure (the three-dimensional structure that allows the function as a vaccine antigen) and the vector is an inactivated vector capable of immunity induction in the same manner as live viruses without use of an adjuvant or with addition of a reduced amount of an adjuvant.

The present inventors have reported that they successfully constructed the type that takes an antigen on a vector by fusion with/substitution by TM and CT sequences of a membrane protein working as a packaging signal to the antigen and a virus (WO 2014/103310). This method enabled efficient expression of an antigen with about 120 amino acid residues. It was difficult to transfer an antigen of 130 or more amino acid residues while maintaining a desired three-dimensional structure.

For example, a sufficient amount of virus can be recovered in a strain with two tandemly linked M2e, but in a strain where a gene with three tandemly linked M2e is cloned into a NotI restriction enzyme site upstream of NP gene of a plasmid for hPIV2 construct, the amount of recovered virus is insufficient to provide an animal test. In the case that RSV F protein gene with a large molecular weight is introduced, recovered viruses show a low titer and viruses having a sufficient titer for practical use as a vaccine cannot be recovered.

In such a virus expression system, a foreign antigenic gene fused with the TM and CT sequences of hPIV2 F protein is inserted into recombinant non-transmissible hPIV2 to load a foreign antigen on the vector particle. It is expected that in the case a high level of expression of antigenic protein, hPIV2 F protein having the same TM and CT sequences becomes competitive in assembling virus particles through M protein and inhibits efficient virus formation, and thus, recovery of viruses with a higher titer is difficult. In fact, it has been reported that when the Ebola virus GP protein of which CT sequence was replaced by that of PIV3 F (TM sequence remains the same as for Ebola virus GP) is transferred into F and HN genes-defective PIV3 vector, the recovery efficiency of PIV3 virus was extremely poor (Non Patent Literature 6). Therefore, it has been considered that when a large foreign antigen having TM and CT sequences is transferred into hPIV2/ΔF by substituting TM and CT sequences of the foreign antigen with TM and CT sequences of hPIV2 F, the recovery of viruses would be more difficult. Further, in the case of PIV3 wherein an antigenic gene was transferred into a site located 5' upstream of NP gene having the highest level of transcription, a mutation was found in the transferred antigenic gene, and this is pointed out as a problem when a foreign gene as an antigen is transferred into a site where it is expressed at a higher level than the virus constituting gene group (Non Patent Literatures 8 and 9). The inventors have also confirmed that when influenza virus M2 gene was introduced into a site 5' upstream of hPIV2 NP gene and viruses were recovered, a mutation was found in M2 gene of the recovered viruses and an amino acid substitution occurred.

In the present invention, an MluI restriction enzyme cleavage site was introduced into an F gene-defective site of an F gene-defective hPIV2 vector, thereby an antigenic gene was introduced into an F gene-defective site immediately 5' upstream of HN gene, and a macromolecular antigen of 130 or more amino acid residues was successfully expressed. As shown specifically in the following examples, the inventors transferred: a gene wherein 3×M2e was fused with TM sequence and/or CT sequence gene of F protein gene of a hPIV2 vector; a plurality of RSV F protein genes wherein the extramembranous sequence domain of RSV F protein gene was intact or modified; and further, RSV F protein gene wherein TM sequence and CT sequence genes of these RSV F proteins were substituted by hPIV2 TM and/or CT sequences, and made a comparison in terms of the recovery level of virus vector and the expression level thereof. Further, regarding RSV F mutants wherein a Fibritin trimerization sequence or a GCN trimerization sequence is inserted, or a nucleic acid encoding a gene wherein an amino acid is substituted by Cys for S-S bond formation enabling the stabilization of trimerization, the inventors are investigating the effects thereof.
Further, the inventors transferred genes wherein four modifications were added to Ebola virus GP protein gene, and Ebola GP gene wherein TM sequence and CT sequence genes of the above four GP genes were substituted by hPIV2 TM and CT sequence gene, and made a comparison in terms of the recovery level of virus vector and the expression level thereof. Also, the inventors transferred: a gene encoding malaria CSP protein; and a gene encoding CSP protein wherein GPI anchor-like sequence of CSP protein was substituted by hPIV2 TM and CT sequences, and the recovery level of vector and the expression of antigen were investigated. Further, the inventors transferred: a gene encoding Zika virus prM/E protein (partially including a mutant); and a gene encoding prM/E protein wherein stem sequence and TM sequence of prM/E protein were substituted by hPIV2 TM and CT sequences, the recovery level of vector and the expression level of antigen were investigated. In this way, the inventors confirmed the effects of the present invention.

"The virus of the family Paramyxoviridae" used in the present invention is particularly preferably "human parainfluenza virus type 2" having monocistronic negative-sense single-stranded RNA of approximately 15,000 bases as the genome. NP protein, P (phospho) protein, M (matrix) protein, F (fusion) protein, HN (hemagglutinin-neuraminidase) protein, and L (large) protein are encoded in this order as viral structural gene products on the genome. In addition, V protein is produced by RNA editing. The nucleocapsid protein (NP) is bound with the RNA genome to form a helically symmetric ribonucleoside-protein complex (nucleocapsid, RNP). Of the proteins encoded on the virus genome, the NP protein, the P (phospho) protein, and the L (large) protein are necessary for the formation of RNP. The F (fusion) protein and the HN (hemagglutinin-neuraminidase) protein reside on the virus envelope and are responsible for adsorption and fusion to a receptor. The M (matrix) protein interacts with the intracytoplasmic domains of the F and HN proteins, the envelope lipid bilayer, and RNP and is important for the budding of virus particles.

Since "human parainfluenza virus type 2 (hPIV2)" is an RNA virus that multiplies in cytoplasm, its gene is not integrated into the chromosomes of host cells. This virus is known to infect the mucous membrane of the human respiratory tract and induce mucosal immunity consisting of IgA mainly and humoral immunity mediated by IgG and cellular immunity. No serious case of infection of humans (adults) by this virus has been previously reported. The virus is therefore considered to be very useful as a virus vector for treatment.

In the present invention, "F gene-defective Paramyxoviridae virus" is used. Preferably, "F gene-defective (non-transmissible) human parainfluenza virus type 2" is used. F protein of human parainfluenza virus type 2 is a protein that is needed for fusing a virus envelope with a cell membrane and transferring virus nucleocapsid into a host. F gene-defective human parainfluenza virus type 2 is a non-transmissible virus that produces infective hPIV2 carrying F gene in packaging cells, but does not produce infective virus in cells without expressing F protein. Thus, this virus cannot constitute a virus particle having the ability to multiply autonomously after infection of cells of a subject and does not infect the other cells. The virus is therefore highly safe as a virus for vaccines.

In order to prepare the F gene-defective human parainfluenza virus type 2 vector, an F gene-defective virus is cultured in cells expressing hPIV2 F protein. The F protein can thereby be retained on the virus envelope in the presence of the F protein supplied in trans from the cells to produce virus particles having infectious ability.

In a preferred embodiment of the present invention, Vero cells are used as the packaging cells that can produce virus particles by infection by the F gene-defective virus. The Vero cells are particularly highly permissive with the human parainfluenza virus type 2 F protein. In addition, these cells do not express interferons, and can stably grow and can efficiently produce virus particles, even if the human parainfluenza virus type 2 F gene is constantly expressed.

In a preferred embodiment of the present invention, the virus vector of the present invention has undergone "nucleic acid inactivation treatment." "Nucleic acid inactivation treatment" refers to the inactivation of only the virus genome in the state where the three-dimensional structures of envelope proteins such as F protein and HN protein and a transferred antigenic polypeptide are maintained and the transferred antigenic polypeptide has neutralizing antibody-inducing ability, which requires the function and the three-dimensional structure of these envelope proteins. The nucleic acid inactivation treatment can be carried out by, for example, nucleic acid-alkylating agent treatment, hydrogen peroxide treatment, UV irradiation, exposure to radiation, or heat treatment. Particularly preferably, the virus vector is treated with a nucleic acid-alkylating agent β-propiolactone. The β-propiolactone can be added to the virus culture solution and incubated therewith at 4°C for approximately 24 hours. The concentration of the β-propiolactone is preferably 0.001% to 0.05%, more preferably 0.004% to 0.01%, in terms of final concentration.

In the virus vector of the present invention, an antigenic gene is integrated on a vector, and this eliminates the need of transferring the antigen after the above nucleic acid inactivation and can circumvent problems associated with the transfer of the antigen after inactivation (low antigen transfer efficiency, disruption of envelope and antigen structures, etc.). Further, by use of the F gene-defective virus, the virus lacks the ability to multiply and thus cannot multiply in a recipient even if the live virus remains after the drug treatment; thus, the high safety of the inactivated vaccine can be kept.

The "virus vector" means a virus particle by which a gene to be expressed in an infected cell, is packaged together with the virus genome, and a vector whose virus genome is not capable of producing a virus having infectious ability. In the present specification, the latter is particularly referred to as an "inactivated vector" and can be obtained by inactivating the nucleic acid by treatment with a drug or the like.

The "antigenic polypeptide" refers to a polypeptide of 130 or more amino acid residues, which can cause immune response in a subject having received administration of the virus vector of the present invention. Examples thereof include influenza, RS virus, Ebola virus, Zika virus or a portion thereof, protozoan proteins or a portion thereof, and bacterium- or virus-derived proteins or a portion thereof. For example, the antigenic polypeptide useful for a influenza vaccine is influenza virus HA protein, NA protein, M2 protein, or M2e protein, or a fragment thereof. In addition, the antigenic polypeptide useful for a RSV vaccine is RSV F protein or G protein or a fragment thereof; the antigenic polypeptide useful for an Ebola virus vaccine is Ebola virus GP protein or a fragment thereof; the antigenic polypeptide useful for a Zika virus vaccine is prM/E protein or a fragment thereof; and the antigenic polypeptide useful for a plasmodial vaccine is CSP protein or a fragment thereof. As long as the antigenic polypeptide has immunogenic enough to function as a vaccine, it may be a fragment as described above and may include a proper mutation. Examples of such mutants include: mutant RSV F protein wherein p27 of RSV F protein or p27 and adjacent sequence is deleted and the cleavage by furin is removed (including a mutant wherein Asn(N) at position 61 and Ser(S) at position 251 are substituted by Ile(I) and Pro(P) (Non Patent Literature 4)); mutant RSV F protein wherein a portion of fusion domain sequence of RSV F protein is deleted (including a mutant wherein Ser(S) at positions 155 and 290 are substituted by Cys(C) (Non Patent Literature 7)); RSV F mutant wherein a Fibritin trimerization sequence of T4 phage, a GCN trimerization sequence, or a nucleic acid encoding a gene wherein an amino acid is substituted by Cys for formation of S-S bond for stabilizing trimerization; mutant GP protein wherein Phe(F) at position 88 and Phe(F) at position 535 in the amino acid sequence (GenBank Accession No. KJ660346) of Ebola virus GP protein are both substituted by Ala(A); mutant GP1 protein wherein a mucin domain sequence located at position 313 and onward from the mutant GP protein is deleted; or mutant GP protein wherein a mucin domain sequence is deleted from the GP sequence.

Examples of infectious disease-related antigenic polypeptides to be transferred include antigenic peptides of viruses such as influenza viruses (including a highly virulent influenza virus), parainfluenza virus type 3, RS virus, Hendra virus, SARS virus, MERS virus, Nipah virus, Lassa virus, dengue virus, West Nile virus, Japanese encephalitis virus, human metapneumovirus, Ebola virus, hantavirus, AIDS virus, hepatitis B virus, hepatitis C virus, rubella virus, rotavirus, norovirus, Crimean-Congo hemorrhagic fever virus, herpesvirus, cytomegalovirus, papillomavirus, Zika virus, Marburg virus, and HIV, and antigenic peptides of bacteria such as the group A beta-hemolytic streptococcus, Mycobacterium tuberculosis, Vibrio cholerae, and mycoplasma, and antigenic peptides of Plasmodium.

In the present invention, the "antigenic polypeptide" is expressed as protein fused with/substituted by TM and/or CT sequences of a vector virus or GPI-like anchor protein on a virus envelope. This allows the antigenic polypeptide expressed in a virus-producing cell to be taken into a virus particle and to be delivered to a recipient. That is, in the present invention, not only the antigenic polypeptide gene integrated into a virus gene is transcribed and translated in an infected cell, but also a virus particle itself has antigenic polypeptide; and this allows a large amount of antigenic polypeptide to be reliably delivered to a recipient.

In order to efficiently express antigenic polypeptide as fusion protein on a virus envelope, a gene encoding antigenic polypeptide is linked to a gene encoding F protein TM and/or CT sequences of a vector virus or a gene encoding GPI-like anchor protein, and thereby integrated into a virus gene. Examples of a gene encoding F protein TM and/or CT sequences of a virus include hPIV2 F gene. Construction of such a vector can be carried out by a standard method using conventional recombinant DNA technique.

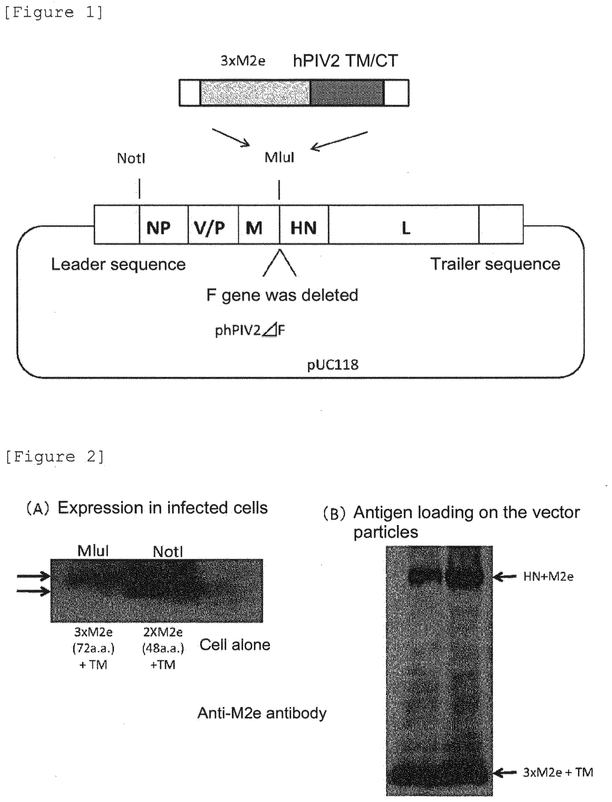

A nucleic acid (gene) encoding antigenic polypeptide is inserted into a F gene-defective site "immediately 5' upstream of HN gene" in a structural gene of the above-mentioned vector virus (see Figure 1). It should be noted that "immediately 5' upstream of HN gene" means immediately upstream of 5'-terminus of NH gene, but a sequence not affecting the expression may be included between the nucleic acid encoding the antigenic polypeptide and 5'-terminus of HN gene. In the present invention, F gene-defective virus originally having the nucleic acid present immediately upstream of HN gene is used, so "immediately 5' upstream of HN gene" can be referred to as an F gene-defective site.

When the protein gene is integrated as a fusion gene or an intact gene into a packaging cell that supplies a structural gene product in trans, a large amount of antigen is expressed on an envelope of defective hPIV2.

The treatment of the virus vector of the present invention with a lower concentration of β-propiolactone than an ordinary concentration can inactivate the genome alone while the virus vector maintains its hemagglutinating activity, cell adsorption ability and ability to maturate dendritic cells (that is, the three-dimensional structure of protein on a vector membrane is maintained in a state similar to that of live viruses). The reason that the treatment can be conducted with β-propiolactone at a lower concentration than usual, that is the lowest concentration that enables the genome to be inactivated is that a non-proliferative (non-transmissible) virus vector that produces no secondary particles is used. Even if a small amount of infective virus remains, no secondary infective particles are produced since the virus is non-transmissible. Regarding the influence to the maturation of dendritic cells by hPIV2 vector through the treatment concentration of β-propiolactone, it has been already reported that the treatment at a lower concentration is advantageous to the maturation of dendritic cells or induction of cytokines (Non Patent Literature 10). Further, many RNA chains having no transcription/replication ability, which are fragmented by the treatment with a low concentration of β-propiolactone, remain in the vector, and natural immunity is activated by the action of RIGI, IFIT, etc. This method has the advantage that: the virus particle itself has adjuvant activity and therefore eliminates the need of combined use with an adjuvant that causes immunity against the antigen or can reduce adjuvant concentration.

The virus vector of the present invention infects a cell via a sialic acid receptor. Since sialic acid resides on the surface of many cells or tissues, the possible administration route of the vector is nasal spray, transpulmonary, oral, sublingual, intradermal, or subcutaneous administration, or direct administration to the vein as well as ex vivo administration to immunity-causing cells such as dendritic cells.

The virus vector of the present invention is useful as a vaccine to a mammal including a human. The vaccine of the present invention includes a virus vector and a pharmaceutically acceptable carrier.

Typically, the vaccine can be administered as an aerosol to cells of a mammal including a human. The aerosol can be prepared by a standard method. For example, a culture supernatant containing the virus vector is concentrated, if necessary, and suspended together with a pharmaceutically acceptable carrier in a buffer solution such as PBS, a virus vector-stabilizing solution, or saline. Then, the suspension can be sterilized by filtration through a filter or the like according to the need and subsequently charged into an aseptic container to prepare the aerosol. The aerosol may be supplemented with a stabilizer, a preservative, and the like, according to the need. The expression vector thus obtained can be administered by inhalation to a subject.

Typically, the virus vector of the present invention can also be administered as an injection (subcutaneous, intradermal, or intramuscular injection) to cells of a mammal including a human. The injection can be prepared by a standard method. For example, a culture supernatant containing the virus vector is concentrated, if necessary, and suspended together with a pharmaceutically acceptable carrier in a buffer solution such as PBS or saline. Then, the suspension can be sterilized by filtration through a filter or the like according to the need and subsequently charged into an aseptic container to prepare the injection. The injection may be supplemented with a stabilizer, a preservative, and the like, according to the need. The expression vector thus obtained can be administered as the injection to a subject.

The amount of vaccine to be administered is from 0.01 µg to 100,000 µg of antigen per ordinary administration, and this is dependent on a subject to be treated, the antibody synthesis ability in the immune system of the subject, and the desired level of prevention, and also dependent on the route of administration such as oral, subdermal, nasal, intradermal, intramuscular or intravenous administration.

The vaccine of the present invention may be provided in a single administration schedule or preferably a multiple administration schedule. In a multiple administration schedule, 1 to 10 separate administrations may be conducted at an initial stage of inoculation, and subsequently, another administration may be conducted at a time interval required to maintain and/or strengthen immune responses, for example, 1 to 4 months later as a second administration. If necessary, administration may be continued a few months later. The regimen is also determined at least partially depending on the necessity of an individual, which depends on the determination of a doctor.

For RSV, Ebola virus, Zika virus and Plasmodium infection, no licensed vaccines have yet to be prepared. Antigens for vaccines are clarified, but the strict three-dimensional structure for unstable antigen is needed for efficient neutralizing antibody induction; and there has been a disadvantage that it was difficult to deliver a macromolecular peptide (several hundred amino acid residues) as an antigen while retaining its three-dimensional structure. With respect to this disadvantage, the virus vector of the present invention can deliver a macromolecular peptide (several hundred amino acid residues) as an antigen while retaining its three-dimensional structure.

Conventionally, such a vector had a disadvantage that it was difficult to recover the virus vector efficiently while the antigenic polypeptide of 130 or more amino acid residues is displayed on a virus particle. According to the present invention, an antigenic polypeptide of 500 or more amino acid residues is displayed on a virus particle to enable efficient recovery of virus vector.

The present invention performs nucleic acid inactivation by use of a low concentration of β-propiolactone or the like, so virus genome alone is inactivated while the three-dimensional structures of virus envelope protein and transferred antigen are maintained. Thus, this vector has cell adsorption properties and immunogenicity similar to those of live viruses, and at the same time, a transferred antigen can maintain the three-dimensional structure of an antigen epitope for inducing an efficient neutralizing antibody, thereby allowing the expectation of an effective vaccine effect.

Regarding the transfer of an antigen, a virus vector with an antigen loaded thereon in advance is produced, and this eliminates the need of transferring the antigen after nucleic acid inactivation and can circumvent problems associated with the transfer of the antigen after inactivation (low antigen transfer efficiency, disruption of envelope and antigen structures, etc. by a conventional method).

From the above advantages, the virus vector of the present invention allows the expectation of excellent effects unlike conventional virus vectors that do not allow the expectation of sufficient effects in a human.

The virus vector of the present invention can be constituted such that the antigen can be located outside or inside, or both outside and inside the vector envelope. This allows a plurality of antigens to be transferred in various manners to vectors.

As described above, the present invention can provide an inactivated vector capable of inducing the immunity almost equal to that of live virus vectors by efficient virus inactivation treatment and delivering an unstable macromolecular antigenic protein while maintaining its structure; and can pave the way for vaccination of inactivated vector by use of the Paramyxoviridae virus.

### Examples

Hereinafter, the present invention will be described in more detail with reference to Examples. However, the present invention is not intended to be limited by these Examples.

### [Example 1]

### Construction of hPIV2/ΔF wherein 3×M2e, and TM sequence and CT sequence of hPIV2 F protein were fused and introduced into an MluI restriction site

At the upstream of a gene encoding TM sequence and CT sequence (N terminus-YSLSAIALILSVITLVVVGLLIAYIIKLVSQIHQFRSLAATTMFHRENPAFFSKNNH GNIYGIS-C terminus) (SEQ ID NO:1): 65 amino acid residues), constructed was a gene, to which a gene having an initiation codon (ATG) linked to a gene encoding a sequence (3×M2e) having three M2e antigenic peptides (N terminus-SLLTEVETPIRNEWGCRCNDSSDD- C terminus (SEQ ID NO: 2)) of a versatile influenza virus; and a gene necessary for expression in hPIV2 virus were added. A plasmid construct of hPIV2/ΔF having MluI restriction enzyme cleavage sequences added to both ends of the constructed gene was constructed (Figure 1). The insertion site is located immediately 5' upstream of HN gene of hPIV2/ΔF. This construct was constructed on the basis of the rule of 6 reportedly important for the construct such that the total number of hPIV2 genomes was a multiple of 6. According to the previous report (Non Patent Literature 11), the virus was recovered by the reverse genetics method. Further, the influenza antigen was transferred into an MluI restriction enzyme cleavage site of hPIV2/ΔF wherein HN protein was fused with an M2e antigen (Figure 1) .

As a result, hPIV2/ΔF wherein a 3×M2e antigenic peptide of versatile influenza virus was fused with TM sequence and CT sequence of hPIV2 F protein was efficiently recovered; M2e was efficiently expressed in infected cells (Figure 2A), and further, it was confirmed that M2e protein was incorporated on vector particles (Figure 2B). When a 3xM2e gene was inserted at a NotI restriction enzyme cleavage site at NP upstream of hPIV2/ΔF, viruses were not recovered efficiently unlike the case where 2×M2e was inserted at NotI restriction enzyme cleavage site, as shown in Figure 2A. When a 3×M2e antigenic gene was inserted into an MluI restriction enzyme cleavage site, even fusion of HN protein with M2e enabled efficient recovery of viruses and allowed an antigen to be incorporated on a vector (Figure 2B).

A vaccine prepared by inactivating an M2e expression vector with a low concentration of β-propiolactone was used, and thereby it was confirmed that an antibody against M2e was induced in mice.

### [Example 2]

### Construction of hPIV2/ΔF wherein an intact F protein gene (codon optimum gene) of RSV or a gene having hPIV2 F protein TM sequence and/or CT sequence replaced from RSV TM sequence and/or CT sequence, and a gene harboring mutations with a higher stability of prefusion F were transferred into an MluI restriction site

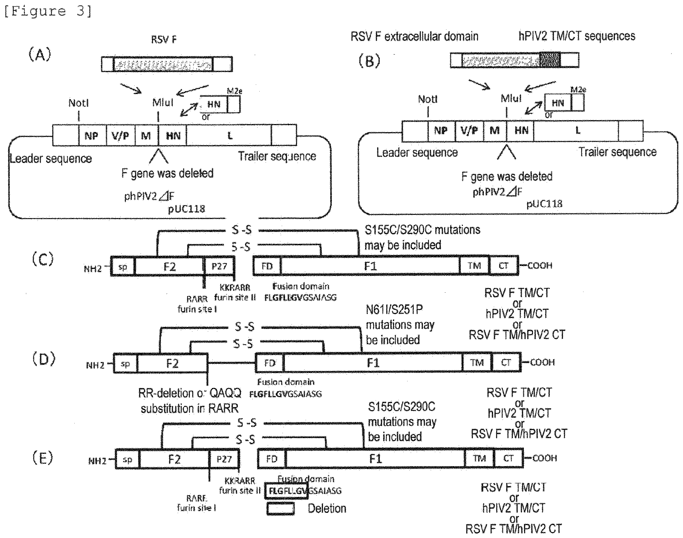

RSV F protein of accession number P03420 was used for an amino acid sequence of an antigen for RSV F vaccine. As a gene encoding the amino acid sequence, RSV F DNA sequence (Catalog No. VG40042-UT) available from Sino Biological Inc. by optimizing an expression codon of each amino acid for humans was used. Transfer was carried out into an MluI restriction sequence of the following plasmid vector for expression of an antigenic gene via hPIV2/ΔF (Figures 3A and 3B). A gene having a gene start sequence, an intervening sequence and a gene end sequence of hPIV2 added to: an intact F protein gene of RSV (Figures 3A and 3C); or a gene (Figures 3B and 3C) encoding a sequence wherein RSV F protein TM and CT sequences (IMITTIIIVIIVILLSLIAVGLLLYCKARSTPVTLSKDQLSGINNIAFSN (SEQ ID NO: 3)) were replaced with hPIV2 TM and CT sequences (YSLSAIALILSVITLVVVGLLIAYIIKLVSQIHQFRSLAATTMFHRENPAFFSKNN HGNIYGIS (SEQ ID NO: 1)) so that RSV F protein is included on hPIV2/DF vector, or RSV F protein CT sequence (KARSTPVTLSKDQLSGINNIAFSN (SEQ ID NO: 9)) was replaced with hPIV2 CT sequence (KLVSQIHQFRSLAATTMFHRENPAFFSKNNHGNIYGIS (SEQ ID NO: 10)), was constructed by a conventional method. Constructs having a partially modified gene start sequence, etc. (a construct which expresses a transferred gene by read-through from the gene without addition of a gene start sequence to 5' upstream of the transferred gene; or a construct which expresses a transferred gene by read-through from the gene without addition of a gene start sequence to 5' upstream of the transferred gene; or a construct wherein two gene start sequences were transferred at 5' upstream of HN gene were also produced. It has been reported that the stability of RSV F mutant is enhanced by replacing Ser(S) at both positions 155 and 290 of RSV F with Cys(C) and producing a S-S bond between cysteines (Reference Literature 7). Thus, F mutant gene having S155C and S290C was produced (Figures 3B and 3C), and transferred into an MluII site of a plasmid vector for hPIV2/ΔF expression. The sequences were inserted on the basis of the rule of 6 (the gene length was a multiple of 6). Further, RSV F mutant wherein a Fibritin trimerization sequence of T4 phage, a GCN trimerization sequence, or a nucleic acid encoding a gene wherein an amino acid is substituted by Cys for formation of S-S bond for stabilizing trimerization was also constructed. According to the previous report (Non Patent Literature 11), the virus was recovered. Viruses of all types provided a cytopathogenic effect (CPE) in infected cells, and viruses with sufficient titers were recovered. According to the previous report (Non Patent Literature 11), the virus was recovered. Viruses of all types provided a cytopathogenic effect (CPE) in infected cells, and viruses with sufficient titers were recovered.

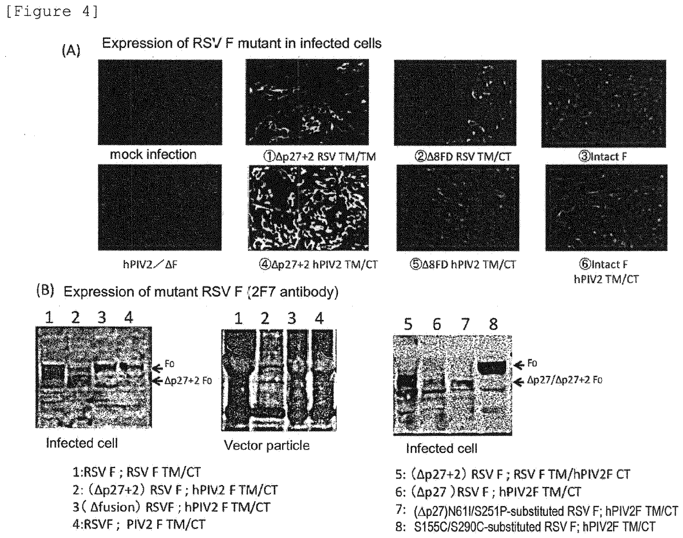

Vero cells expressing F gene were infected by the recovered virus, an antibody (anti F monoclonal antibody [2F7] of RSV available from Abcam plc.) against RSV F was used to confirm RSV F expression by immunostaining. After 2-day infection, fixation was conducted with 100% cold methanol, expression confirmation was conducted with a fluorescence microscope by use of Alexa488-labeled secondary antibodies. In cells expressing hPIV2 F and hPIV2/ΔF not harboring RSV F, no response to an antibody was observed. However, hPIV2/ΔF harboring intact F protein gene of RSV and a RSV F protein gene having hPIV2 F TM sequence and/or CT sequence replaced from RSV F TM sequence and/CT sequence exhibited a positive reaction to an anti F antibody against RSV F (Figure 4(A)). Further, it was confirmed that a protein of interest was expressed by Western blotting analysis using an anti F monoclonal antibody [2F7] (Figure 4(B)).

### [Example 3]

### Construction of hPIV2/ΔF wherein a RSV F protein gene having removal of two furin cleavage domain peptide sequence genes of RSV or a gene having a sequence with hPIV2 F protein TM sequence and/or CT sequence replaced from RSV F protein TM sequence and/or CT sequence was inserted into an MluI restriction site

In RSV F, as shown in Figure 3D, two furin recognition sequence sites are present (Figure 3C). RSV F (FΔp27+2) was constructed, wherein an amino acid (N terminus-ELPRFMNYTLNNAKKTNVTLSKKRKRR-C terminus (SEQ ID NO: 4)) called as p27 between the furin recognition sequences was removed and further (Arg(R) and Arg(R) at 108 and 109) of the forward furin recognition sequence were deleted (Figure 3D). A gene having a gene start sequence, an intervening sequence and a gene end sequence of hPIV2 added to FΔp27+2, or a sequence having a replacement gene (Figures 3B and 3D) encoding a sequence having: hPIV2 TM sequence and CT sequence (YSLSAIALILSVITLVVVGLLIAYIIKLVSQIHQFRSLAATTMFHRENPAFFSKNN HGNIYGIS (SEQ ID NO: 1)) replaced from RSV F protein TM sequence (Figures 3A and 3D) and CT sequence (IMITTIIIVIIVILLSLIAVGLLLYCKARSTPVTLSKDQLSGINNIAFSN (SEQ ID NO: 3)); or hPIV2 CT sequence (KLVSQIHQFRSLAATTMFHRENPAFFSKNNHGNIYGIS (SEQ ID NO: 10)) replaced from RSV F protein CT sequence (KARSTPVTLSKDQLSGINNIAFSN (SEQ ID NO: 9)) so that RSV F protein is contained on a hPIV2/ΔF vector was inserted into an Mlu site of an expression vector hPIV2/ΔF. Further, a mutant (FΔp27: Figure 3D) having deletion of p27 sequence by replacement of the initial furin recognition sequence of RSV F (from the sequence of Arg(R)Ala(A)Arg(R)Arg(R) at positions 106, 107, 108 and 109 to Gln(Q)Ala(A)Gln(Q)Gln(Q)) was also constructed, and a mutant gene having hPIV2 TM sequence and/or CT sequence replaced from RSV F protein TM sequence and/or CT sequence was inserted into an MluII site of an expression vector hPIV2/ΔF. The purpose of production of these mutants is to attempt the stabilization of the three-dimensional structure of prefusion F by introducing mutations, to induce an antibody with a high neutralizing activity to a prefusion antigen, and to develop an RSV vaccine with high efficacy. It was reported that replacements in RSV F of Asn(N) at position 67 and Ser(S) at position 251 with Ile(I) and Pro(P), respectively, enhanced the stability of RSV F mutant (Non Patent Literature 4). A mutant gene of FΔp27 harboring N61I and S251P (Figures 3B and 3D) was produced and was inserted into an MluI site of an expression plasmid vector hPIV2/ΔF. The sequences were inserted on the basis of the rule of 6. According to the previous report (Non Patent Literature 11), the virus was recovered. Further, a RSV F mutant harboring a nucleic acid encoding: a Fibritin trimerization sequence of T4 phage; a GCN trimerization sequence; or a gene having Cyc replaced from an amino acid for formation of S-S bond for stabilizing trimerization was also prepared. Viruses of all types provided a cytopathogenic effect (CPE) in infected cells, and viruses with sufficient titers were recovered.

Vero cells expressing F gene were infected by the recovered virus, an antibody (anti F monoclonal antibody [2F7] of RSV available from Abcam plc.) against RSV F was used to confirm RSV F expression by immunostaining. After 2-day infection, fixation was conducted with 100% cold methanol, expression confirmation was conducted with a fluorescence microscope by use of Alexa488-labeled secondary antibodies. RSV FΔp27+2 protein gene, and hPIV2/ΔF harboring RSV FΔp27+2 protein gene with hPIV2 F TM sequence and/or CT sequence replaced from RSV FΔp27+2 protein exhibited a positive reaction to an anti F antibody against RSV F (Figure 4(A)). The expression of a protein of interest was confirmed by Western blotting analysis using an anti F monoclonal antibody [2F7] (In #5 of Figure 4(B), results obtained by inserting RSV F TM/PIV2 F CT of FΔp27 into TM and CT sequences are shown).

### [Example 4]

### Construction of hPIV2/ΔF wherein a F protein gene having removal of eight amino acids of RSV membrane fusion sequence, a gene encoding TM sequence and/or CT sequence of the RSV F protein gene, or a gene having a sequence with hPIV2 F protein TM sequence and CT protein gene replaced from a gene having a mutation for a high stability of prefusion F was inserted into an MluI restriction site

In RSV F, as shown in Figure 3C, the sequence of amino acids from positions 137 to 151 (FLGFLLGVGSAIASG (SEQ ID NO: 5) is called as fusion domain (FD) and is a domain involved in fusion of cells, etc. In this example, RSV F protein having removal of eight amino acids (FLGFLLGV (SEQ ID NO: 6) from the RSV F protein was constructed (Figure 3E). The reason that a portion of the FD sequence was removed for construction was that since hPIV2/ΔF lacks an F gene, no proliferative virus is produced other than in an F expression packaging cells; however, it has been reported that RSV F protein alone is possibly adhered to and fused with a cell, and in order to ensure the safety, a portion of the fusion sequence was removed from FD to eliminate the possibility. A gene having a gene start sequence, an intervening sequence and a gene end sequence of hPIV2 added to the RSV F protein (Figures 3A and 3E), or a sequence having a replacement gene (Figures 3B and 3E) encoding a sequence having: hPIV2 TM sequence and CT sequence (YSLSAIALILSVITLVVVGLLIAYIIKLVSQIHQFRSLAATTMFHRENPAFFSKNN HGNIYGIS (SEQ ID NO: 1)) replaced from TM sequence and CT sequence (IMITTIIIVIIVILLSLIAVGLLLYCKARSTPVTLSKDQLSGINNIAFSN (SEQ ID NO: 3)) of the RSV F protein; or hPIV2 CT sequence (KLVSQIHQFRSLAATTMFHRENPAFFSKNNHGNIYGIS (SEQ ID NO: 10)) replaced from RSV F protein CT sequence (KARSTPVTLSKDQLSGINNIAFSN (SEQ ID NO: 9)) was inserted into an Mlu site of an expression plasmid vector hPIV2/ΔF. It was reported that crosslinking of S-S bond between cysteines by replacing Ser(S) with Cyc(C) at both positions 155 and 290 in RSV F enhanced the stability of RSV F mutant (Non Patent Literature 7). An F mutant gene (Figures 3B and 3E) harboring S155C and S290C was prepared and inserted into an MluII site of an expression plasmid vector hPIV2/ΔF. The sequences were inserted on the basis of the rule of 6. According to the previous report (Non Patent Literature 11), the virus was recovered and the virus recovery was successfully completed. Further, a RSV F mutant harboring a nucleic acid encoding: a Fibritin trimerization sequence of T4 phage; a GCN trimerization sequence; or a gene having Cyc replaced from an amino acid for formation of S-S bond for stabilizing trimerization was also prepared.

Vero cells expressing F gene were infected by the recovered virus, an antibody (anti F monoclonal antibody [2F7] of RSV available from Abcam plc.) against RSV F was used to confirm RSV F expression by immunostaining. After 2-day infection, fixation was conducted with 100% cold methanol, expression confirmation was conducted with a fluorescence microscope by use of Alexa488-labeled secondary antibodies. The protein gene having removal of a partial domain of FD of RSV F, and hPIV2/ΔF harboring RSV FΔp27 protein gene with hPIV2 F TM sequence and CT sequence replaced from TM sequence and CT sequence of the F protein of RSV exhibited a positive reaction to an anti F antibody against RSV F (Figure 4(A)). Further, the expression of a protein of interest was confirmed by Western blotting analysis using an anti F monoclonal antibody [2F7] (Figure 4(B)).

### [Example 5]

### Construction of GP protein gene of Ebola virus harboring two amino acid mutations or hPIV2/ΔF wherein a gene having a sequence with hPIV2 F protein TM sequence and CT sequence replaced from TM sequence and CT sequence of the GP protein gene was inserted into an MluI restriction site

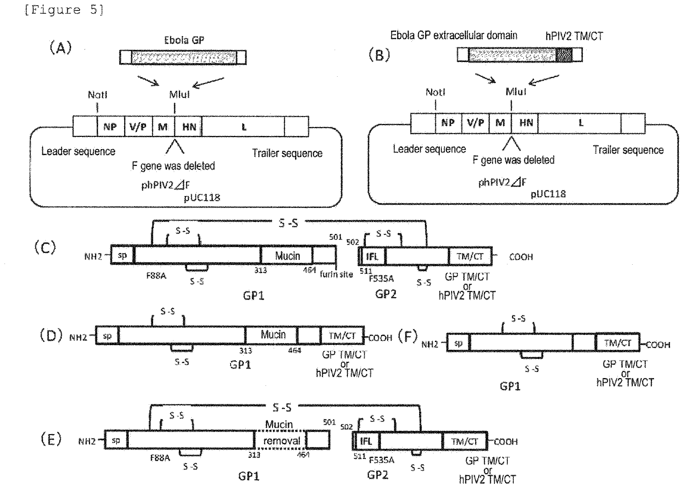

A GP protein of Ebola virus is a protein responsible for binding of a virus to a cell surface receptor and membrane fusion between a viral envelope and host cell membrane (Figure 5C). As cDNA, a commercially available plasmid (Sino Biological Inc., Accession Number: KJ660346, cDNA length: 2031 bp) was used. A partial sequence of the GP protein was replaced with the sequence (F88A and F535A; JVI: 2013 (87) 3324-3334: Non Patent Literature 15) reportedly having no entry capability to cells. Further, a gene replacement (no replacement of amino acid sequence) was carried out so that RNA editing would not occur. A gene having a gene start sequence, an intervening sequence and a gene end sequence of hPIV2 added to the GP protein gene (Figures 5A and 5C) of Ebola virus, or a sequence having hPIV2 TM sequence and CT sequence (YSLSAIALILSVITLVVVGLLIAYIIKLVSQIHQFRSLAATTMFHRENPAFFSKNN HGNIYGIS (SEQ ID NO: 1)) replaced from TM sequence and CT sequence (WIPAGIGVTGVIIAVIALFCICKFVF (SEQ ID NO: 7)) of the GP protein gene, was inserted into an Mlu site of an expression plasmid vector hPIV2/ΔF. The sequences were inserted on the basis of the rule of 6. According to the previous report (Non Patent Literature 11), the virus was recovered.

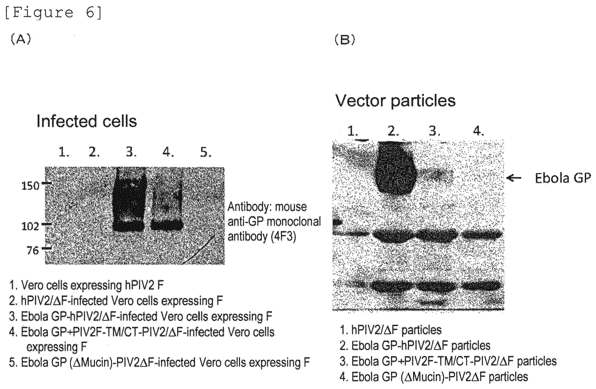

In all cases, viruses were successfully recovered. Vero cells expressing PIV2 F gene were by the recovered viruses for 2 days, and the expression of GP was confirmed (Figure 6). For the confirmation of the GP protein expression, anti GP mouse monoclonal antibody (4F3: IBT BIOSEVICES) was used. In infected cells, the GP protein and GP protein replaced by hPIV2 TM sequence and CT sequence were expressed at the same level (Figure 6A). GP protein having removal of a mucin domain described below made no reaction. (An epitope sequence of that antibody was not specified, but it was expected that an epitope of the mucin domain was recognized since the GP has no mucin domain.)

Next, viruses were recovered from virus expressing cell supernatant by ultracentrifugation at 141,000xg for 35 minutes, GP protein contained in a vector was checked by Western blotting (WB) (Figure 6B). As a result, contrary to the initial expectation, it was found that more GP protein carrying TM sequence and CT sequence derived from Ebola GP protein was incorporated on the vector in comparison with GP protein having replaced with hPIV2 TM sequence and CT sequence (Figure 6B) (both had the same level of expression of NP and P of PIV2). The WB results of infected cells in Figure 6 show that the GP proteins (GP protein before glycosylation with ER and Golgi body) near 102 kDa had similar GP expression levels when TM/CT sequences of either of GP and hPIV2 F were carried. However, it was found that regarding 4F3 positive GP protein (glycosylated GP protein) with a higher molecular weight, one carrying TM/CT sequences of Ebola virus GP had a higher level of expression. It was confirmed that the GP protein having replaced with hPIV2 F TM/CT sequences was subjected to glycosylation and incorporated on a vector. As an exemplary virus where a protein carrying TM/CT sequences different from its original is incorporated in, Ebola GP protein (Non Patent Literature 6), VSV G protein (Non Patent Literature 12) and others are reported.

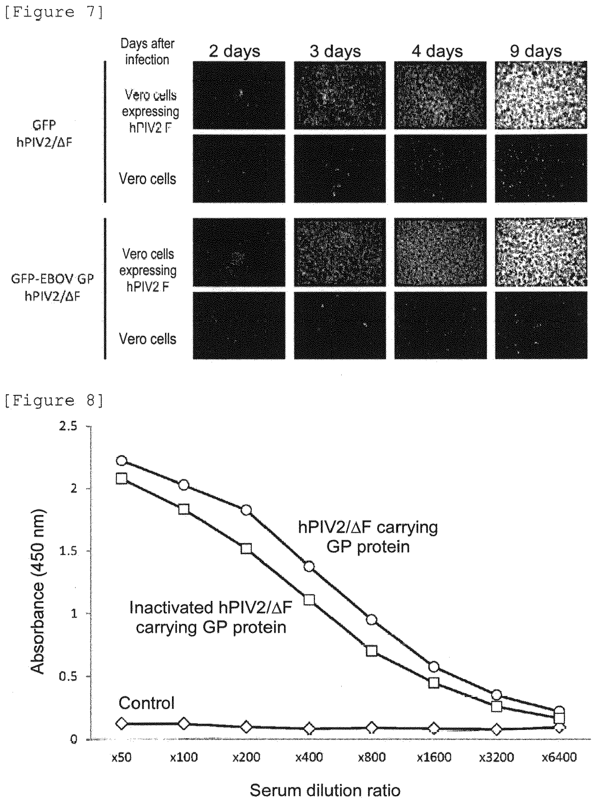

As described above, it was found that the incorporation efficiency of the Ebola GP protein into hPIV2/ΔF was extremely high. With the expectation of future practical application, in order to confirm that hPIV2/ΔF carrying Ebola GP protein (the above-described two amino acid replacements) does not proliferate in normal cells, a plasmid wherein a GFP gene expression sequence was inserted into an NotI restriction enzyme cleavage site upstream of NP gene of a hPIV2/ΔF plasmid containing the Ebola GP gene was constructed and the virus was prepared. Vero cells and Vero cells constitutively expressing PIV2 F were infected by the virus and an hPIV2/ΔF vector carrying only a GFP gene, respectively, so that they had an MOI of 0.01 (Figure 7). Just like the hPIV2/ΔF carrying GFP gene, which was confirmed that no infective particles appeared in cells other than Vero cells expressing hPIV2 F, no infective particles of the virus were found in Vero cells not expressing hPIV2 F and proliferation of infective particles were found only in Vero cells expressing hPIV2 F. That is, it was confirmed that the virus did not produce infective particles in normal cells and had a high level of safety.

### [Example 6]

### Construction of GP1 protein gene wherein GP1 protein gene of Ebola virus was fused with TM sequence and CT sequence gene of Ebola virus GP or hPIV2/ΔF wherein a gene having GP1 protein sequencing gene of Ebola virus fused with TM sequence and CT sequence of hPIV2 F protein was inserted into an MluI restriction site

A GP1 protein gene of Ebola virus was constructed, in which TM and CT sequences (WIPAGIGVTGVIIAVIALFCICKFVF (SEQ ID NO: 7)) (Figures 5A and 5D) of a GP protein gene or hPIV2 TM and CT sequences (YSLSAIALILSVITLVVVGLLIAYIIKLVSQIHQFRSLAATTMFHRENPAFFSKNN HGNIYGIS (SEQ ID NO: 1)) (Figures 5B and 5D) were added to a GP1 protein (GP1 protein domain obtained by cleaving a GP1 protein into GP1 and GP2 with furin) (furin cleaved site eliminated) of the forward furin cleavage recognition sequence of the GP protein gene of Ebola virus constructed in Example 5. A gene having a gene start sequence, an intervening sequence and a gene end sequence of hPIV2 added thereto was inserted into an Mlu site of an expression plasmid vector hPIV2/ΔF. The sequences were inserted on the basis of the rule of 6. According to the previous report (Non Patent Literature 11), the virus was recovered.

### [Example 7]

### Construction of GP having removed a mucin domain gene from Ebola virus GP protein harboring two amino acid mutations or hPIV2/ΔF wherein a gene having a sequence with hPIV2 F protein TM and CT sequences replaced from TM and CT sequences of the GP protein gene was inserted into an MluI restriction site

A domain from positions 313 to 454 of amino acid sequence of Ebola virus GP is a sequence called as a mucin domain, and a mucin domain protein is arranged in the form of covering a domain formed by other domain and has many glycosylated amino acid residues. It is known as a domain that is not much involved in adsorption or entry to cells of GP protein, and GP (Δmucin GP) of Ebola virus having removed this domain was constructed. A gene having a gene start sequence, an intervening sequence and a gene end sequence of hPIV2 added to a sequence having replaced to TM and CT sequences (YSLSAIALILSVITLVVVGLLIAYIIKLVSQIHQFRSLAATTMFHRENPAFFSKNN HGNIYGIS (SEQ ID NO: 1)) (Figures 5B and 5E) of Ebola virus F in the Δmucin GP (Figures 5A and 5E) was inserted into an Mlu site of an expression plasmid vector hPIV2/ΔF. The sequences were inserted on the basis of the rule of 6. According to the previous report (Non Patent Literature 11), the virus was recovered, and viruses in all cases were successfully recovered. Then, Western blotting analysis was conducted. However, anti GP antibodies used herein are highly likely to have a mucin domain as an epitope (Figure 6), and whether the expression occurred or not was not analyzed by Western blotting analysis (NP and P of hPIV2 were expressed).

### [Example 8]

### Construction of gene having removed a mucin domain gene from Ebola virus GP1 protein harboring two amino acid mutations, or hPIV2/ΔF wherein a gene with a sequence having hPIV2 F protein TM and CT sequences replaced from TM and CT sequences of the GP1 protein gene was inserted into an MluI restriction site

A GP1 protein gene of Ebola virus was constructed by removing a mucin coding region sequence from the GP1 protein gene of Ebola virus constructed in Example 5 and adding TM and CT sequences (WIPAGIGVTGVIIAVIALFCICKFVF (SEQ ID NO: 7))of a GP protein gene (Figures 5A and 5F) or hPIV2 TM and CT sequences (YSLSAIALILSVITLVVVGLLIAYIIKLVSQIHQFRSLAATTMFHRENPAFFSKNN HGNIYGIS (SEQ ID NO: 1)) (Figures 5B and 5F) to the sequence, and a gene to which a gene start sequence, an intervening sequence and a gene end sequence of hPIV2 were added was inserted into an Mlu site. The sequences were inserted on the basis of the rule of 6. According to the previous report (Non Patent Literature 11), the virus was recovered.

### [Example 9]

### Evaluation on antibody titer against GP protein in mice by hPIV2/ΔF carrying Ebola virus GP protein gene harboring two amino acid mutations constructed in Example 5 and an inactivated vector by treating the vector with 0.01% β-propiolactone.

The vector and inactivated vector (adjuvant was not added) were prepared so that they had a virus number of 10⁸ /mL. 100 µl of respective vector samples and a control sample were injected into the femoral muscle of the hind limb of 5-week-old BALB/c mice (female) twice at an interval of two weeks, and sera were recovered two weeks after the administration. The sera were used, and an increase of antibodies (IgGl + IgG2a) was evaluated by ELISA using Ebola virus GP protein (Sino biological Inc. cat#: 40442-V08H2). 50 ng of GP protein was applied to one well of a 96-well plate, and the above sera were diluted from 50-fold to 6400-fold by two times, and the presence of antibody was evaluated by a normal ABC method. As shown in results of Figure 8, it was confirmed that hPIV2/ΔF carrying Ebola virus GP protein gene and an inactivated vector by treating the vector with 0.01% β-propiolactone both effectively increased IgG antibodies against Ebola virus GP. It was also confirmed that antibodies having a neutralizing activity were induced.

### [Example 10]

### Construction of hPIV2/ΔF carrying malaria antigen CSP (circumsporozoite protein; plasmodial sporozoite surface protein)

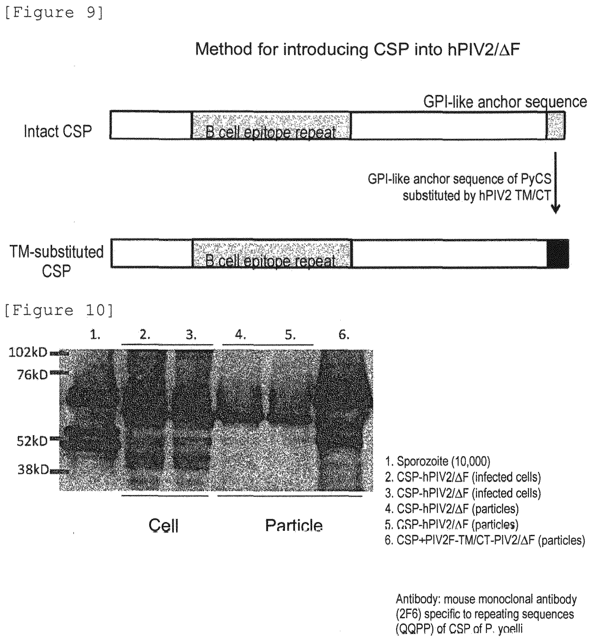

CSP derived from Plasmodium yoelli was inserted into hPIV2/ΔF. A CSP gene was prepared by replacing an intact CSP gene and a GPI-like anchor sequence (CSSIFNIVSNSLGFVILLVLVFFN (SEQ ID NO; 8)) with hPIV2 CT sequence (YSLSAIALILSVITLVVVGLLIAYIIKLVSQIHQFRSLAATTMFHRENPAFFSKNN HGNIYGIS (SEQ ID NO: 1)), and a sequence necessary for hPIV2 expression was added to the CSP gene. The resultant CSP gene was inserted into an MluI restriction enzyme site of an expression plasmid vector hPIV2/ΔF (Figure 9). Viruses were recovered according to the previous report (Non Patent Literature 11), and viruses for both were successfully recovered. Vero cells expressing F were infected by the viruses, and the expression of CSP protein was analyzed by Western blotting analysis. As an antibody against CSP, a monoclonal antibody against QQPP repeat of Plasmodium yoelli was used (2F6). As a positive control, 10,000 sporozoites of Plasmodium yoelli were used.

As shown in Figure 10, CSP protein was expressed by the vector. In general, it has been reported that the expression of CSP protein was not easy. However, hPIV2 enabled CSP protein to be expressed easily. Therefore, it is expected to be used as means for expressing other malaria related genes. Also, it is expected to be used as means for expressing other malaria related genes. Further, it was confirmed that virus vectors recovered from a culture supernatant by ultracentrifugation (141,000 g for 35 minutes) can carry intact CSP carrying a GPI-like anchor sequence and CSP having hPIV2 TM replaced from the GPI-like anchor sequence on hPIV2/ΔF vector particles at the same level. Regarding CSP protein, it was understood that GPI-like anchor protein had an ability to carry CSP protein on a vector at the same level as hPIV2 TM protein. However, with respect to the transmembrane domain, CSP of the original GPI was different from CSP having replaced to hPIV2 TM/CT in terms of the molecular weight reactive with an antibody against a QQPP antigen. The CSP having replaced to hPIV2 TM/CT exhibited the same molecular weight as sporozoite CSP protein of positive control, but the CSP of original GPI exhibited a different molecular weight of the positive band. The CSP having replaced to hPIV2 TM/CT was taken in a vector in a form similar to that of the original (sporozoite CSP)

### [Example 11]

### Evaluation on antibody titer against CSP by hPIV2/ΔF carrying malaria antigen CSP

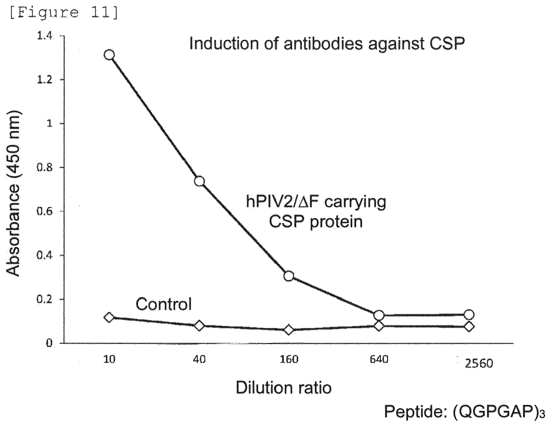

hPIV2/ΔF carrying a CSP gene having hPIV2 TM replaced from a GPI-like anchor sequence was prepared so that the virus number was 10⁹ /mL. 100 µl of respective vector samples and a control sample were injected into the femoral muscle of the hind limb of 5-week-old BALB/c mice (female) twice at an interval of two weeks, and sera were recovered two weeks after the administration. The sera were used to evaluate an antibody titer against CSP of Plasmodium yoelli. For the evaluation by ELISA, a (QGPGAP)₃ peptide having three repeating QGPGAP sequences of a B cell epitope repeatedly present in CSP of Plasmodium yoelli was used. The peptide was prepared so that it had 5 µg/mL and applied to one well of a 96-well plate overnight, and an increase of antibodies (IgG1 + IgG2a) against CSP was evaluated. The above sera were diluted from 10-fold to 2560-fold by four times, and the presence of antibody was evaluated by a normal ABC method. Results (Figure 11) show that administration of the vector increased antibodies against CSP.

### [Example 12]

### Preparation of Zika virus vaccine using prM/E antigen

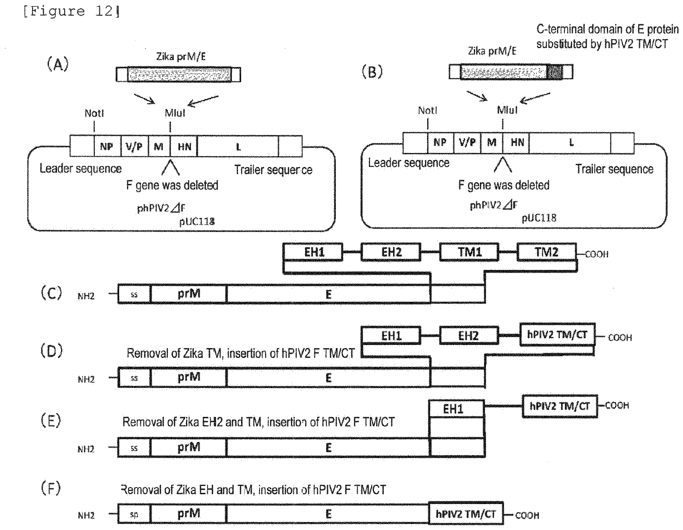

A gene prepared by adding an initiation codon was added to 5'-terminus of prM gene in a gene (partially including amino acid mutation) encoding prM/E domain (KU321639) of Zika virus, adding a gene having an addition or no addition of a gene encoding a secretion signal, and adding a gene start sequence, an intervening sequence and a gene end sequence of hPIV2 before and after the gene, and inserted into an MluI site of an expression plasmid vector hPIV2/ΔF (Figures 12A and 12C). The sequences were inserted on the basis of the rule of 6. According to the previous report (Non Patent Literature 11), the virus was recovered.

### [Example 13]

### Preparation of vaccine having a substitution or a deletion of stem sequence (EH1 and EH2) and TM sequence of C-terminus region of Zika virus E protein

In Zika virus prM gene having the same structure as in Example 14, a gene encoding a protein, in which hPIV2 TM/CT sequences (TM sequence and/or CT sequence of hPIV2 (YSLSAIALILSVITLVVVGLLIAYIIKLVSQIHQFRSLAATTMFHRENPAFFSKNN HGNIYGIS (SEQ ID NO: 1)) was added to E protein having a deletion of the following sequence, was constructed. The following sequence mentioned herein signifies EH1 and EH2 sequences and TM1 and TM2 sequences, EH2 sequence and TM1 and TM2 sequences, or TM1 and TM2 sequences of E protein. A gene having a gene start sequence, an intervening sequence and a gene end sequence of hPIV2 added before and after the gene was prepared, and inserted into an MluI site of an expression plasmid vector hPIV2/ΔF (Figures 12B, 12D, 12E and 12F). The sequences were inserted on the basis of the rule of 6. According to the previous report (Non Patent Literature 11), the virus was recovered.

### [Example 14]

### Effect of vaccine against Zika virus

The vector carrying prM/E or E protein, which was produced in Example 15, was inactivated with a low concentration of β-propiolactone, and the thus prepared vector was used to evaluate a vaccine effect against Zika virus.

### [Example 15]

### Recovery of prM/E protein or M/E protein granules (subviral particles) not including genetic information

Regarding viruses belonging to Flavivirus, it has been reported that other flaviviruses release not only infectious virus particles from ER, but also protein granules (subviral particles) of prM/E protein or M/E protein not containing genetic information (Non Patent Literatures 13 and 14). A vector having a prM/E gene of Zika virus inserted into non-transmitting hPIV2/ΔF was prepared (Figures 12A and 12C). Cells (Vero cells, CHO cells, etc.) not expressing F gene of hPIV2 were infected by the vector, the granules were effectively recovered and used as virus-like particles (VLP) for Zika vaccine.

### Industrial Applicability

A virus vector of the present invention can present a macromolecular antigenic peptide (having several hundred amino acid residues) on a virus particle in a state its three-dimensional structure is maintained. The virus vector of the present invention has a cell adsorption property and immunogenicity similar to those of live viruses even after nucleic acid inactivation treatment and presents an antigenic epitope while its three-dimensional structure is maintained, and it can induce an effective neutralizing antibody. Thus, the present invention is useful for production of a licensed vaccine effective to RSV, Ebola virus, etc.

## Claims

1. A non-transmissible virus vector in which a nucleic acid encoding an antigenic polypeptide is inserted immediately 5' upstream of HN gene of an F gene-defective Paramyxoviridae virus gene, wherein the antigenic polypeptide is expressed as a fusion protein of 130 or more amino acid residues, fused with a TM sequence and/or a CT sequence derived from the virus.

2. The virus vector according to claim 1, wherein the Paramyxoviridae virus is a non-transmissible human parainfluenza virus type 2.

3. The virus vector according to claim 1 or 2, wherein the antigenic polypeptide is expressed on the surface of a vector envelope.

4. The virus vector according to any one of claims 1 to 3, wherein the antigenic polypeptide is expressed while maintaining a natural three-dimensional structure or a three-dimensional structure required as a vaccine antigen.

5. The virus vector according to any one of claims 1 to 4, wherein the virus has undergone nucleic acid inactivation treatment.

6. The virus vector according to any one of claims 1 to 5, wherein a TM sequence and/or a CT sequence of an antigenic polypeptide gene or a GPI-like anchor protein is integrated as a nucleic acid substituted by a TM sequence and/or a CT sequence derived from human parainfluenza virus type 2.

7. The virus vector according to any one of claims 1 to 6, wherein the antigenic polypeptide is any one or more of:
an antigenic peptide of a virus selected from influenza viruses including a highly virulent influenza virus, parainfluenza virus type 3, RS virus, Hendra virus, SARS virus, MERS virus, Nipah virus, Lassa virus, dengue virus, West Nile virus, Japanese encephalitis virus, human metapneumovirus, Ebola virus, hantavirus, AIDS virus, hepatitis B virus, hepatitis C virus, rubella virus, rotavirus, norovirus, Crimean-Congo hemorrhagic fever virus, herpesvirus, cytomegalovirus, Zika virus, Marburg virus, HIV and papillomavirus;
an antigenic peptide of a bacterium selected from the group A beta-hemolytic streptococcus, Mycobacterium tuberculosis, Vibrio cholerae, and mycoplasma;
an antigenic peptide of plasmodium; and
an antigenic peptide selected from the group consisting of cancer antigens gp100, MUC1, NY-ESO-1, MelanA/MART1, TRP2, MAGE, CEA, CA125, HER2/neu, WT1, PSA and a neoantigen,
or fragment(s) thereof.

8. The virus vector according to claim 7, wherein the antigenic polypeptide is any one selected from: an antigen having three or more consecutive M2e of influenza virus; RSV F protein or G protein, or a mutant thereof or a fragment thereof; M2e antigen and RSV F protein or a fragment thereof; and Ebola virus GP protein, or a mutant thereof or a fragment thereof; and malaria CSP protein, or a mutant thereof or a fragment thereof; and Zika virus prM/E protein, or a mutant thereof or a fragment thereof.

9. A vaccine comprising the virus vector according to any one of claims 1 to 8 and a pharmaceutically acceptable carrier.


SEQUENCE LISTING

<110>  Mie university
       BioComo Inc.

<120>  F gene-defective human parainfluenza type II virus vector and
       vaccine

<130>  PMU-9004WO

<150>  JP2015-119097
<151>  2015-06-12

<150>  JP2016-063315
<151>  2016-03-28

<160>  10

<170>  PatentIn version 3.5

<210>  1
<211>  64
<212>  PRT
<213>  Human parainfluenza virus 2

<400>  1

Tyr Ser Leu Ser Ala Ile Ala Leu Ile Leu Ser Val Ile Thr Leu Val
1               5                   10                  15

Val Val Gly Leu Leu Ile Ala Tyr Ile Ile Lys Leu Val Ser Gln Ile
            20                  25                  30

His Gln Phe Arg Ser Leu Ala Ala Thr Thr Met Phe His Arg Glu Asn
        35                  40                  45

Pro Ala Phe Phe Ser Lys Asn Asn His Gly Asn Ile Tyr Gly Ile Ser
    50                  55                  60

<210>  2
<211>  24
<212>  PRT
<213>  Influenza A virus

<400>  2

Ser Leu Leu Thr Glu Val Glu Thr Pro Ile Arg Asn Glu Trp Gly Cys
1               5                   10                  15

Arg Cys Asn Asp Ser Ser Asp Asp
            20

<210>  3
<211>  50
<212>  PRT
<213>  respiratory syncytial virus

<400>  3

Ile Met Ile Thr Thr Ile Ile Ile Val Ile Ile Val Ile Leu Leu Ser
1               5                   10                  15

Leu Ile Ala Val Gly Leu Leu Leu Tyr Cys Lys Ala Arg Ser Thr Pro
            20                  25                  30

Val Thr Leu Ser Lys Asp Gln Leu Ser Gly Ile Asn Asn Ile Ala Phe
        35                  40                  45

Ser Asn
    50

<210>  4
<211>  27
<212>  PRT
<213>  respiratory syncytial virus

<400>  4

Glu Leu Pro Arg Phe Met Asn Tyr Thr Leu Asn Asn Ala Lys Lys Thr
1               5                   10                  15

Asn Val Thr Leu Ser Lys Lys Arg Lys Arg Arg
            20                  25

<210>  5
<211>  15
<212>  PRT
<213>  respiratory syncytial virus

<400>  5

Phe Leu Gly Phe Leu Leu Gly Val Gly Ser Ala Ile Ala Ser Gly
1               5                   10                  15

<210>  6
<211>  8
<212>  PRT
<213>  respiratory syncytial virus

<400>  6

Phe Leu Gly Phe Leu Leu Gly Val
1               5

<210>  7
<211>  26
<212>  PRT
<213>  respiratory syncytial virus

<400>  7

Trp Ile Pro Ala Gly Ile Gly Val Thr Gly Val Ile Ile Ala Val Ile

1               5                   10                  15

Ala Leu Phe Cys Ile Cys Lys Phe Val Phe
            20                  25

<210>  8
<211>  24
<212>  PRT
<213>  Plasmodium yoelii 17X

<400>  8

Cys Ser Ser Ile Phe Asn Ile Val Ser Asn Ser Leu Gly Phe Val Ile
1               5                   10                  15

Leu Leu Val Leu Val Phe Phe Asn
            20

<210>  9
<211>  24
<212>  PRT
<213>  respiratory syncytial virus

<400>  9

Lys Ala Arg Ser Thr Pro Val Thr Leu Ser Lys Asp Gln Leu Ser Gly
1               5                   10                  15

Ile Asn Asn Ile Ala Phe Ser Asn
            20

<210>  10
<211>  38
<212>  PRT
<213>  respiratory syncytial virus

<400>  10

Lys Leu Val Ser Gln Ile His Gln Phe Arg Ser Leu Ala Ala Thr Thr
1               5                   10                  15

Met Phe His Arg Glu Asn Pro Ala Phe Phe Ser Lys Asn Asn His Gly
            20                  25                  30

Asn Ile Tyr Gly Ile Ser
        35